# EUROPEAN PATENT APPLICATION

(11) **EP 4 644 566 A1**
(43) Date of publication of application: **05.11.2025**
(21) Application number: 22969814.7
(22) Date of filing: 30.12.2022
(51) Int. Cl.: C12Q 1/68, C12Q 1/6874, C12Q 1/6869, C12Q 1/6837

(54) **IMPROVED NUCLEIC ACID CAPTURE METHOD**

(71) Applicant: BGI Shenzhen, Shenzhen, Guangdong 518083 (CN); BGI Shenzhen Co., Ltd, Shenzhen, Guangdong 518083 (CN)
(72) Inventor: ZHAO, Yu, Shenzhen, Guangdong 518083 (CN); JIANG, Xia, Shenzhen, Guangdong 518083 (CN); YUAN, Qiaomei, Shenzhen, Guangdong 518083 (CN); LIAO, Sha, Shenzhen, Guangdong 518083 (CN); CHEN, Ao, Shenzhen, Guangdong 518083 (CN); ZHANG, Wenwei, Shenzhen, Guangdong 518083 (CN)
(74) Representative: Lawrie IP Limited
(86) International application number: PCT/CN2022/144104
(87) International publication number: WO 2024/138672

(57) **Abstract**

An improved nucleic acid capture method, comprising the following steps: by means of a splint oligonucleotide hybridization random probe and an oligonucleotide strand fixed on a capture chip, an RNA in a sample captured by the random probe undergoing reverse transcription into a cDNA, and linking the cDNA to the oligonucleotide strand, the reverse transcription and the linking being carried out in the same reaction system. During the process, while the RNA undergoes the reverse transcription into the cDNA, the cDNA is linked, by means of splint hybridization of fixed oligonucleotide sequences at two ends, to the oligonucleotide strand fixed on the chip. The method can increase the number of captured genes, and reduce the loss of cDNA; in addition, the method combines what was originally three steps into one step, thus further shortening the duration of the process.

## Description

### TECHNICAL FIELD

The present disclosure belongs to the field of genetics, and specifically relates to an improved nucleic acid capture method.

### BACKGROUND

Cells within an organism function in their respective spatial locations, and the gene expression of each cell is closely associated with the physiological or pathological processes which it regulates. Spatial transcriptomic technology enables the analysis of *in situ* expression of genes in tissues, thereby enabling the study of developmental processes and disease progression.

The spatial transcriptomic technology currently used involves reverse transcribing an RNA captured in tissues into a stable cDNA, followed by *in situ* ligation of the cDNA to a capture chip, so as to be able to fix the spatial location of the cDNA, thereby determining gene expression at a certain spatial location through sequencing.

However, the number of genes captured by this method remains far below expectations, with many genes being undetected, which results in inaccuracies in spatial transcriptomic analysis, failing to reflect the true gene expression in tissues. There is thus an urgent need for a method capable of capturing more genes to more accurately reflect the true gene expression in tissues.

### SUMMARY

The present disclosure primarily addresses the issue of cDNA loss in the prior art by providing an improved nucleic acid capture method. Specifically, the present disclosure combines reverse transcription (RT) and T4 ligation into a single step, during which RT enzyme and T4 ligase are added simultaneously, and the reaction is carried out at 37°C for 3 to 5 hours. During the process, while RNA is reverse transcribed into cDNA, the cDNA is ligated, by means of splint hybridization of fixed oligonucleotide sequences at both ends, to an oligonucleotide strand fixed on the chip. The method can significantly increase the number of captured genes, reduce the loss of cDNA, and further significantly shorten the duration of the process.

Tissue sections (frozen or paraffin-embedded samples) are first mounted on the surface of a capture chip, followed by fixation and permeabilization (paraffin-embedded samples are first deparaffinized and decrosslinked).

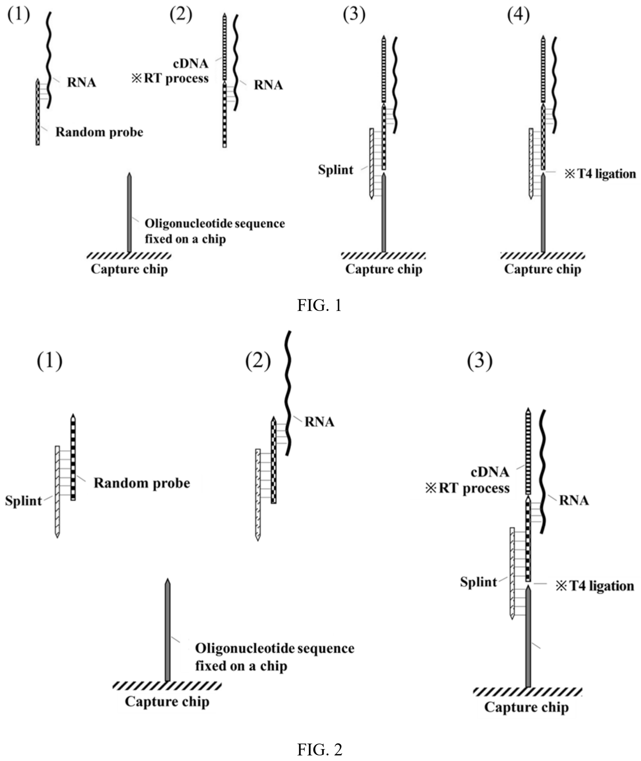

Concurrent with tissue fixation, a random probe such as 6N, and a splint are hybridized in 5× SSC buffer at 55°C for 10 minutes ((1) in FIG. 2).

After tissue permeabilization, the hybridized primer hybridization mixture is applied to the chip surface and hybridized in 5× SSC at room temperature for 15 to 30 minutes, during which the random probe such as 6N in the primer hybridization mixture captures RNA in the tissue ((2) in FIG. 2).

Subsequently, a reaction mixture of RTase and T4 ligase is applied to the chip surface and reacted at 37°C for 3 to 5 hours, enabling simultaneous RT and T4 ligation, i.e., the cDNA is fixed to the capture chip via T4 ligation during RT ((3) in FIG. 2).

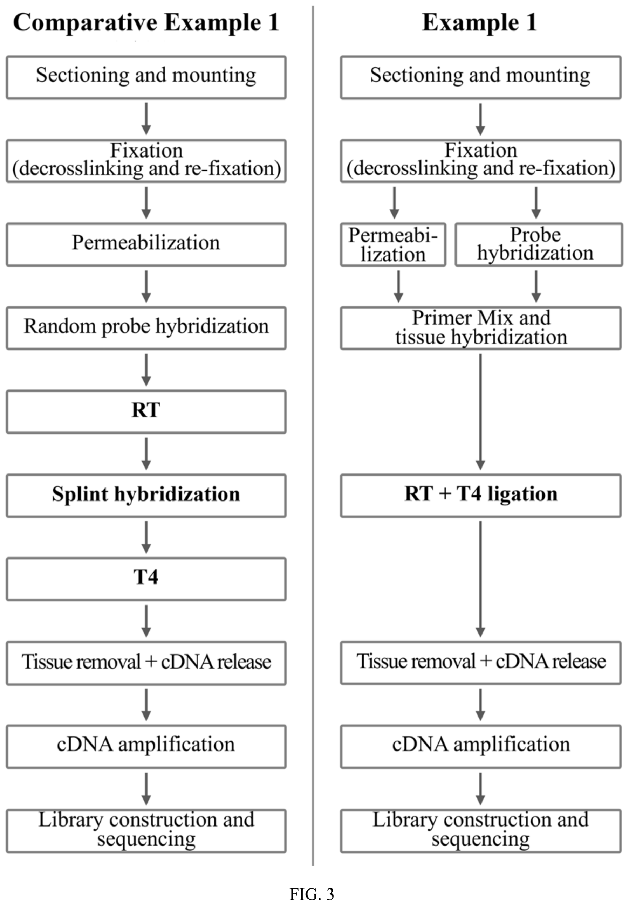

The operational flowcharts for the method of the present disclosure (simultaneous RT and T4 ligation) and the method of Comparative Example 1 (RT followed by T4 ligation) are shown in FIG. 3. The operational steps for the method of Example 1 are detailed as follows (with reference to FIGs. 2 and 3): Fresh tissue-embedded blocks are sectioned, and the sections are mounted on a capture chip, followed by baking at 37°C for 3 minutes (paraffin-embedded tissue sections are deparaffinized and decrosslinked). The tissue-mounted chip is then fixed in methanol at -20°C for 30 minutes. The subsequent steps are the same for both types of tissue sections: Concurrent with tissue fixation in methanol, a random probe (containing 6N) with a fixed oligonucleotide sequence at the 5' end, and a splint are mixed at a molar ratio of 1:1 in 5× SSC containing RNase inhibitor (RI) to prepare a mixture with a final concentration of 0.1 µM. The mixture is hybridized at 55°C for 10 minutes to allow hybridization between the splint and the fixed oligonucleotide sequence at the 5' end of the random probe, resulting in a hybridization probe. After fixation in methanol, the tissue-mounted chip is removed and permeabilized at 37°C for 20 minutes. The chip is then removed and washed once with 5× SSC containing RI. The hybridized mixture of random probes and splints is added to allow hybridization between the random probe such as 6N and RNA in the tissue at room temperature for 15 minutes. The chip is then washed once with 5× SSC containing RI to remove random probes not hybridized to RNA. The liquid is aspirated from the chip surface, and a premix containing reverse transcriptase (RT enzyme) and T4 ligase is added. The reaction is carried out at 37°C for at least 3 hours (up to overnight). After completion of the reaction, the chip surface is washed with nuclease-free water, and then tissue removal solution is added. Tissue removal is performed at 55°C for 10 minutes (20 minutes for paraffin-embedded tissues). After tissue removal, cDNA release mixture is added, and the reaction is carried out at 55°C for 3 hours to release cDNA into the solution. The obtained cDNA is then purified using magnetic beads and subjected to PCR amplification to generate cDNA libraries. 20 ng of cDNA libraries is fragmented using Tn5 transposase, resulting in sequencing libraries for second-generation high-throughput sequencing.

The method provided by the present disclosure combines reverse transcriptase and T4 ligase in the same reaction system. During the process, while RNA is reverse transcribed into cDNA, the cDNA is ligated, by means of splint hybridization of the random probe and an oligonucleotide strand on the chip, to the oligonucleotide strand fixed on the chip. The method can significantly increase the number of captured genes, reduce the loss of cDNA, and further significantly shorten the duration of the process.

To address the shortcomings of the prior art, the first aspect of the present disclosure provides a method for capturing nucleic acid, comprising the following steps:
reverse transcribing an RNA in a sample captured by a random probe into a cDNA, and ligating the cDNA to an oligonucleotide strand by means of splint oligonucleotide hybridization of a random probe and an oligonucleotide strand fixed on a capture chip, wherein the reverse transcription and the ligation are carried out in the same reaction system.

In some examples, the method comprises the following steps:
(1) hybridizing of the random probe to the splint sequence; wherein the 5' end of the random probe is partially complementary to the 5' end of the splint sequence;
(2) hybridizing of the random probe to the RNA in the sample; wherein the 3' end of the random probe is partially complementary to the 3' end of the RNA;
(3) hybridizing of the splint sequence to the oligonucleotide sequence;
   wherein steps (1), (2), and (3) have no temporal sequence and can be performed simultaneously in the same reaction system.
   preferably, the oligonucleotide sequence is fixed on the capture chip;
(4) in the same reaction system, synthesizing cDNA using the RNA in the complex formed by simultaneous hybridization in steps (1), (2), and (3) as a template, and ligating the fixed oligonucleotide sequence to the random probe in the complex, allowing the oligonucleotide sequence, the random probe, and the cDNA to form a long single-stranded nucleotide sequence containing cDNA in the 5' to 3' direction.

The steps (3) and (4) have no temporal sequence and can also be performed simultaneously.

In some examples, in step (1), the random probe comprises 6 to 20N;
in step (3), the capture chip is reacted at 30 to 45°C, such as 37°C, for 3 to 24 hours, such as 3 to 5 hours;
in step (4), a reverse transcriptase and a DNA ligase are present simultaneously in the reaction system.

Preferably, the sample comprises a tissue sample.

In some examples, the step further comprises:
(5) aspirating the liquid from the surface of the capture chip, adding a mixture 1 containing a reverse transcriptase and a T4 ligase, and reacting the capture chip in an incubator for 3 to 24 hours; the T4 ligase is, for example, a thermostable T4 ligase or a high-salt-tolerant T4 ligase.

In some embodiments, the mixture 1 contains a reverse transcription reagent, 3 to 25 mM of MgCl₂, 1 to 50 mM of DTT, and 1 mM or more of ATP;
the volume ratio of the reverse transcriptase to T4 ligase is 2:1 or 1:1; and/or,
the incubator has a temperature of 35 to 42°C; the reaction can be appropriately prolonged at a low temperature, for example, the reaction can be carried out at 35°C for 24 hours.

In some embodiments, the mixture has a pH of 8.3.

The optimal reaction buffer for reverse transcriptase contains 3 mM of MgCl₂, 10 mM of DTT, pH 8.3 at 25°C, with an optimal reaction temperature of 42°C; the optimal reaction buffer for T4 ligase contains 10 mM of MgCl₂, 1 mM of ATP, 10 mM of DTT, pH 7.5 at 25°C, with an optimal reaction temperature of 16°C. During the experimental design, taking into account the differences in the optimal reaction buffers and reaction temperatures for the two enzymes, the capture at various buffer compositions and reaction temperatures are tested. It is found that using the reaction buffer for reverse transcriptase supplemented with 1 mM of ATP at a reaction temperature of 37°C can achieve good capture efficiency.

In some examples, before step (5), the method further comprises:
(i) while fixing a tissue section to the capture chip, preparing a probe hybridization solution and mixing with 5× SSC, performing a hybridization in an incubator at 50 to 60°C, such as 55°C, for 5 to 30 minutes, such as 10 minutes, to form a primer hybridization mixture, removing and cooling the primer hybridization mixture, and adding 2 to 15% by volume, such as 5% by volume of RNase inhibitor;
(ii) removing the capture chip and aspirating the liquid from the chip surface, washing the capture chip with a mixture 2, adding the primer hybridization mixture to the chip surface, performing a hybridization at room temperature for 15 to 60 minutes, and washing the capture chip again with a mixture 2;

the mixture 2 contains 5× SSC and RNase inhibitor;
the probe hybridization solution contains the random probe and splint.

The steps (i) and (ii) are before the step (5) and after the step (4).

In some examples, the tissue section is a frozen OCT-embedded tissue section;
the probe hybridization solution contains the random probe and splint with a molar ratio of 1:1;
the cooling involves placing the mixture on ice and/or placing the mixture at room temperature until the mixture cools to room temperature, and/or,
the volume ratio of 5× SSC to RNase inhibitor in the mixture 2 is (5 to 30):1, such as 19:1.

In some examples, the sequence of the random probe is as shown in SEQ ID NO: 2; the sequence of the splint is as shown in SEQ ID NO: 3.

In some examples, the method further comprises the following steps:
(a) after completing step (ii), removing the tissue sample from the capture chip;
(b) recovering cDNA from the capture chip; optionally further comprising the steps of cDNA amplification and purification.

In some examples, the capture chip comprises a capture probe, wherein the capture probe comprises spatial barcode location information and a fixed oligonucleotide sequence; the fixed oligonucleotide sequence is hybridized to the 3' end of the splint. Furthermore, the fixed oligonucleotide sequence may be ligated to the 5' end of the random probe.

In some examples, the spatial barcode location information is Nm, the fixed oligonucleotide sequence is as set forth in SEQ ID NO: 1, and the capture probe is 5'-Nm-SEQ ID NO: 1-3', wherein m is 8 to 30.

In some examples, the m is 25 or more.

The capture chip further comprises a fixed oligonucleotide sequence' for PCR amplification and Read 1 sequencing of a cDNA library.

The second aspect of the present disclosure provides a high-throughput sequencing method, comprising the following steps:
(I) obtaining a long single-stranded nucleotide containing cDNA to be sequenced according to the method as described in the first aspect of the present disclosure;
(II) constructing a cDNA library;
(III) sequencing the library and analyzing the obtained data.

In some examples, step (I) is followed by the steps of cDNA fragmentation, amplification, and purification; and/or, in step (II), the cDNA library is circularized to obtain a circularized library of DNB.

On the basis of common sense in the art, the above preferred conditions can be combined arbitrarily to obtain various preferred examples of the present disclosure.

The reagents and raw materials used in the present disclosure are commercially available.

The positive and progressive effects of the present disclosure are as follows:

The method of the present disclosure combines reverse transcriptase and T4 ligase in the same reaction system. During the process, while RNA is reverse transcribed into cDNA, the cDNA is ligated, by means of splint hybridization of the random probe and the oligonucleotide strand on the chip, to the oligonucleotide strand fixed on the chip. The method combines the original three steps into one step, thereby increasing the number of captured genes, reducing the loss of cDNA, and further significantly shortening the duration of the process.

### BRIEF DESCRIPTION OF THE DRAWINGS

FIG. 1 shows a schematic diagram of the principle of spatial transcriptomic technology based on capture of RNA by a random probe in Comparative Example 1.
FIG. 2 shows a schematic diagram of the principle of the improved RT and T4 ligation method of the present disclosure (the arrow direction indicates the 3' end of the oligonucleotide strand).
FIG. 3 shows a comparison of operational flowcharts between Comparative Example 1 (RT followed by T4 ligation) and Example 1 (RT + T4 ligation).
FIG. 4 shows a schematic diagram of the principle of the T4 ligation followed by RT method.
FIG. 5 shows a schematic diagram of hybridization between the splint alone and the oligonucleotide strand on the chip.
FIG. 6 shows a comparison of gene capture efficiency between the RT + T4 ligation method of Example 1 and the RT followed by T4 ligation method of Comparative Example 1.
FIG. 7 shows the capture efficiency of the T4 ligation followed by RT method of Comparative Example 2 and the variable temperature hybridization method of Example 4.

### DETAILED DESCRIPTION OF THE PREFERRED EMBODIMENT

### Example 1: RT + T4 ligation method

### 1.1 Sectioning of frozen samples

The cryostat chamber was pre-cooled to -20°C and the specimen head was pre-cooled to -10°C to -15°C, and experimental tools such as brushes and blades were pre-cooled in the chamber at -20°C in advance. Frozen OCT (optimal cutting temperature compound)-embedded tissue blocks were removed from a -80°C freezer and equilibrated in the cryostat, then fixed onto the specimen holder using liquid-state OCT at room temperature, and frozen until the OCT solidified. The tissue block was trimmed with a pre-cooled blade as needed, followed by sectioning.

### 1.2 RNA quality control

10 to 20 tissue sections of 10 µm thickness were cut and placed into a 1.5 mL EP tube pre-chilled to -20°C. Total RNA was extracted using the RNeasy Mini Kit (QIAGEN) and RNA quality was assessed using the Agilent RNA 6000 Pico Kit (Agilent). Only samples with an RNA integrity number (RIN) of 7 or more were eligible for subsequent experiments.

### 1.3 Chip processing and tissue mounting

The capture chip was carefully gripped with forceps and placed into a new 24-well plate. The capture chip contained a capture probe comprising spatial barcode location information and a fixed oligonucleotide sequence: 5'-N₂₅-TTGTCTTCCTAAGACCGCTTGG-3' (the underlined portion corresponds to SEQ ID NO: 1, and N₂₅ represents 25 consecutive Ns). The chip was washed twice with 400 µL of 0.1× SSC, followed by thorough washing with nuclease-free water (NF-H₂O), and dried at 37°C. A frozen OCT-embedded tissue section of 10 µm thickness was cut and flattened. The capture chip was gripped with forceps and swiftly brought into contact with the frozen section, allowing the tissue section to adhere to the chip. The tissue-mounted chip was then dried at 37°C for 3 to 5 minutes.

### 1.4 Fixation and permeabilization of tissue sections

The dried chip was fixed in a methanol solution pre-chilled to -20°C for 30 minutes. After removal, methanol was air-dried, and permeabilization reagent (prepared by mixing permease [Stereo-seq Transcriptome Kit T, Cat. No.: 101KT114; PR Enzyme 1000028500, PR Rinse Buffer 1000033684] with 10 µL of 0.1 N HCl and 90 µL of NF-H₂O) was added. The chip was reacted in an incubator at 37°C for 10 to 13 minutes.

### 1.5 Primer hybridization mixture preparation and tissue hybridization

A probe hybridization solution was prepared while fixing the tissue section: 5 µM random probe (6N) and 5 µM splint were added to 5× SSC, mixed well, and hybridized in an incubator at 55°C for 10 minutes to form a primer hybridization mixture ((1) in FIG. 2). The mixture was removed and placed on ice, and 5% by volume of RNase inhibitor (RI) was added. The random probe sequence was 5'-CCTCCGACTGTGTGACTTAGACTTGCACTTGATGTGCTNNNNNN-3' (SEQ ID NO: 2), and the splint sequence was 5'-CAAGTGCAAGTCTAAGTCACACAGTCGGAGGCCAAGCGGTCTTAG-3' (SEQ ID NO: 3). After completion of the previous step, the chip was removed, and the liquid was aspirated from the chip surface. The chip was washed once with a mixture solution of 190 µL 5× SSC and 10 µL RI, added with the prepared primer hybridization mixture, and hybridized at room temperature for 30 minutes. The chip was then washed once with 5× SSC and RI.

### 1.6 Reverse transcription (RT) and ligation with T4 ligase

The liquid was aspirated from the chip surface, and a mixture of RTase and T4 ligase (containing RT reagent, 3 mM of MgCl₂, 10 mM of DTT, 1 mM of ATP, pH 8.3 at 25°C; 2.5 µL/ 100 µL of RT enzyme and 5 µL/ 100 µL of T4 ligase) was added. The chip was reacted in an incubator at 37°C for 3 to 5 hours.

### 1.7 Tissue removal

After completion of the reaction, the chip was removed, and the liquid was aspirated from the chip surface. The chip was then washed once with NF-H₂O. Tissue removal solution was added, and the chip was reacted in an incubator at 55°C for 10 minutes. After removal of the chip, the chip surface was pipetted up and down, followed by pipetting up and down twice with NF-H₂O to completely remove the tissue.

### 1.8 cDNA recovery, amplification, and purification

1.8.1 cDNA recovery: 400 µL/well of cDNA release solution was added to the reaction wells of the chip, which were sealed with sealing film. The chip was covered with a lid, and the edges were secured to prevent volatilization. The chip was reacted in an incubator at 55°C for 3 hours to release cDNA. The liquid from the reaction wells was transferred to a new 1.5 mL centrifuge tube. The chip was then rinsed with 350 µL/well of NF-H₂O, and the rinse solution was collected into the same centrifuge tube to obtain a recovery solution. VAHTS^{™} DNA Clean Beads (VAZYME) were mixed with the recovery solution at a ratio of 0.8:1 in the 1.5 mL centrifuge tube. The mixture was shaken and mixed well, and incubated at room temperature for 10 minutes. After a brief centrifugation, the centrifuge tube was placed on a magnetic rack for 3 minutes until the solution was clarified, and the supernatant was discarded. 1 mL of freshly prepared 80% ethanol was added. The mixture was shaken and mixed well, followed by a brief centrifugation, and allowed to stand for 30 seconds. The supernatant was discarded, and the centrifuge tube was washed again with 80% ethanol. The supernatant was discarded, and the mixture was air-dried at room temperature until the surface of the magnetic beads was free of reflection and showed no cracks. 42 µL of NF-H₂O was added for re-dissolution. The mixture was shaken and mixed well, and allowed to stand at room temperature for 5 minutes. After a brief centrifugation, the centrifuge tube was placed on a magnetic rack for 3 to 5 minutes until the solution was clarified, and the supernatant was transferred to a new PCR tube.

1.8.2 cDNA amplification: 58 µL of PCR mixture (containing 50 µL of cDNA HIFI Master Mix and 8 µL of cDNA Primers) was added to the PCR tube, totaling 100 µL, followed by PCR reaction. The steps of PCR reaction were as follows: i) 95°C for 5 minutes; ii) 98°C for 20 seconds; iii) 58°C for 20 seconds; and iv) 72°C for 3 minutes. Steps ii) to iv) were repeated for 15 cycles, followed by 72°C for 5 minutes. The mixture was removed and placed on ice to obtain a PCR product. The amplified cDNA concentration was assayed using Qubit dsDNA Mix containing 198 µL of Invitrogen^{™} Qubit dsDNA HS Buffer, 1 µL of Qubit dsDNA HS Reagent (200×), and 1 µL of cDNA product.

1.8.3 cDNA purification: The PCR product from the previous step was transferred to a new 1.5 mL centrifuge tube, and mixed with VAHTS^{™} DNA Clean Beads (VAZYME) equilibrated at room temperature at a volume ratio of 1:0.6. The mixture was shaken and mixed well, and incubated at room temperature for 10 minutes. After a brief centrifugation, the centrifuge tube was placed on a magnetic rack for 3 minutes until the solution was clarified, and the supernatant was discarded. 1 mL of freshly prepared 80% ethanol was added, and the mixture was allowed to stand for 30 seconds. The supernatant was discarded, and the centrifuge tube was washed again with 80% ethanol. The supernatant was discarded, and the mixture was air-dried at room temperature until the surface of the magnetic beads was free of reflection and showed no cracks. 40 µL of NF-H₂O was added for re-dissolution. The mixture was shaken and mixed well, and allowed to stand at room temperature for 5 minutes. After a brief centrifugation, the centrifuge tube was placed on a magnetic rack for 3 to 5 minutes until the solution was clarified, and the supernatant was transferred to a new 1.5 mL centrifuge tube to obtain a cDNA purification product. 1 µL of cDNA sample was tested for concentration using Qubit dsDNA HS Kit, and cDNA fragment distribution was analyzed using Agilent 2100 High Sensitivity DNA Kit. (QC criteria: Fragment size is mainly distributed in 1000 to 1500 bp.)

### 1.9 cDNA fragmentation, amplification, and purification

1.9.1 cDNA fragmentation: A fragmentation mixture was prepared by mixing 4 µL of 5× TAG buffer, 1 µL of transposase, and 20 ng of the cDNA purification product. The mixture was supplemented with NF-H₂O to a total volume to 20 µL, reacted at 55°C for 10 minutes, and immediately placed on ice to obtain a fragmented product. 5 µL of stop buffer was added to the fragmented product, and the mixture was allowed to stand at room temperature for 5 minutes.

1.9.2 Amplification of fragmented product: 50 µL of Library HIFI Master Mix and 25 µL of Library PCR Primer Mix were added to the reaction mixture, mixed well, followed by PCR reaction. The steps of PCR reaction were as follows: i) 95°C for 5 minutes; ii) 98°C for 20 seconds; iii) 58°C for 20 seconds; and iv) 72°C for 30 seconds. Steps ii) to iv) were repeated for 13 cycles, followed by 72°C for 5 minutes. The mixture was removed and placed on ice to obtain an amplified product.

1.9.3 Purification of amplified product: 100 µL of the amplified product was mixed with 60 µL of VAHTS^{™} DNA Clean Beads (VAZYME) equilibrated at room temperature. The mixture was shaken and mixed well, and incubated at room temperature for 5 minutes. After a brief centrifugation, the tube was placed on a magnetic rack for 3 minutes until the solution was clarified, and the supernatant was transferred to a new PCR tube. 20 µL of Vazyme Beads was added to the supernatant. The mixture was shaken and mixed well, and incubated at room temperature for 5 minutes. After a brief centrifugation, the tube was placed on a magnetic rack for 3 to 5 minutes until the solution was clarified, and the supernatant was discarded. 200 µL of freshly prepared 80% ethanol was added, and the mixture was allowed to stand for 30 seconds. The supernatant was discarded, and the tube was washed again with 80% ethanol. The magnetic beads were air-dried at room temperature until the surface of the magnetic beads was free of reflection and showed no cracks. 20 µL of NF-H₂O was added for re-dissolution. The mixture was shaken and mixed well, and allowed to stand at room temperature for 5 minutes. After a brief centrifugation, the tube was placed on a magnetic rack for 3 minutes until the solution was clarified, and the supernatant was transferred to a new centrifuge tube to obtain a purified product. 1 µL of the purified product was tested for concentration using Qubit dsDNA HS Kit, and fragment distribution was analyzed using Agilent 2100 High Sensitivity DNA Kit. (QC criteria: Fragment size is mainly distributed in 400 to 600 bp.)

### 1.10 cDNA library circularization, sequencing, and data analysis

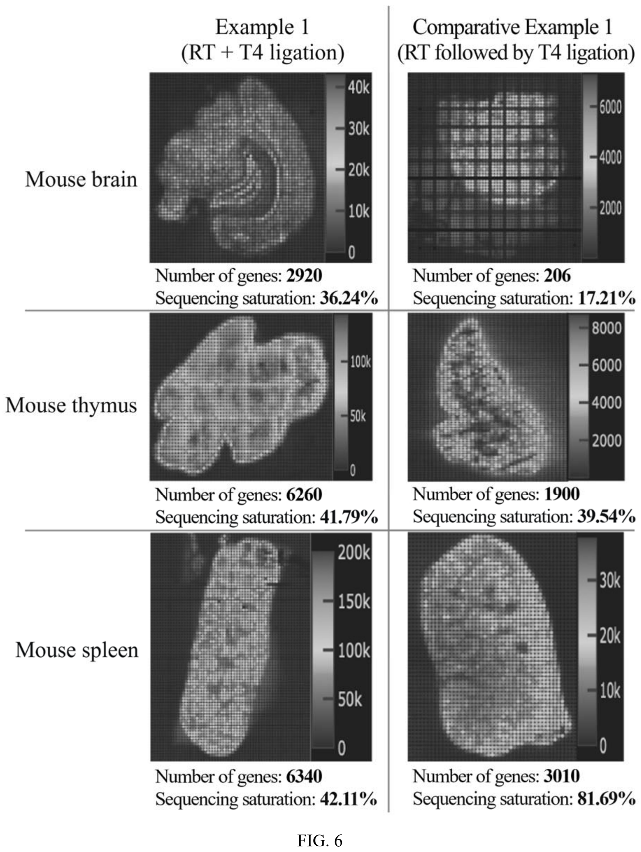

40 ng of the purified product was added to a new PCR tube, supplemented with NF-H₂O to a total volume to 20 µL, and added with 20 µL of 2× Make DNB buffer (corresponding kit for MGISEQ-2000RS). The mixture was reacted in a PCR instrument: 95°C for 3 minutes, and 40°C for 3 minutes. The reaction mixture was removed and placed on ice, followed by the addition of 39 µL of RCA buffer, 1 µL of 10 mM ATP, and 4 µL of One Step Enzyme. The mixture was reacted in the PCR instrument at 30°C for 30 minutes. The reaction mixture was removed and added with 20 µL of DNB stop buffer to obtain a circularized library of DNB. The libraries were sequenced on a MGISEQ-2000RS sequencer. The sequencing data was automatically analyzed on the web page (https://uat.stomics.tech/sap/) to generate heat map results, as shown in the left panel of FIG. 6.

### Comparative Example 1: RT followed by T4 ligation method

Steps 1.1 to 1.4 of Comparative Example 1 were identical to those of Example 1. Steps 1.5 and 1.6 of Comparative Example 1 were as follows:

### 1.5 Random probe 6N hybridization with tissue and reverse transcription (RT)

1.5.1 Random probe 6N hybridization with tissue: 5 µM random probe 6N was dissolved in 5× SSC buffer, added with 5% by volume of RI, and mixed well. The permeabilized tissue section was washed once with 5× SSC and RI, then added with the prepared random probe 6N solution, and hybridized at room temperature for 15 minutes. During the process, RNA in the tissue was captured by random probe 6N. The tissue section was then washed once with 5× SSC and RI.

1.5.2 Reverse transcription (RT) reaction: RT reaction buffer (containing RT reagent, 3 mM MgCl₂, 10 mM DTT, 1 mM ATP, pH 8.3 at 25°C; 2.5 µL/100 µL of RT enzyme) was applied to the chip surface. The chip was reacted at 42°C for 3 hours. After removal, the chip was washed once with 0.1× SSC and RI.

### 1.6 Splint hybridization and T4 ligation

1.6.1 Splint hybridization: 5 µM splint was diluted in 5× SSC buffer, added with 5% by volume of RI, and mixed well. The mixture was added to the section washed in the previous step, and subjected to hybridization at room temperature for 15 minutes.

1.6.2 T4 ligation: T4 ligation reaction buffer (containing 10 mM MgCl₂, 1 mM ATP, 10 mM DTT, pH 7.5 at 25°C) was added, and the mixture was reacted at 16°C overnight.

Steps 1.7 to 1.10 of Comparative Example 1 were identical to those of Example 1. A schematic diagram of the principle of spatial transcriptomic technology based on capture of RNA by random probe in Comparative Example 1 is shown in FIG. 1. A comparison of operational flowcharts between Comparative Example 1 (RT followed by T4 ligation) and Example 1 (RT + T4 ligation) is shown in FIG. 3.

The results were shown in the right panel of FIG. 6. The specific analysis was as follows:
Mouse brain: Half of a mouse brain was used for the experiment. When using the method of Comparative Example 1, the gene capture efficiency in the mouse brain was low. As shown in the figure, at a sequencing saturation of 17%, only 206 gene types were detected per bin 200. In contrast, when using the method of Example 1, at a sequencing saturation of 36%, a total of 2920 gene types were detected per bin 200. According to estimates, even if the sequencing saturation reached 36% when using the method of Comparative Example 1, only approximately 450 gene types would be detected per bin 200. Therefore, by using the method of Example 1, a 6.5-fold improvement in gene capture efficiency in the mouse brain was achieved, demonstrating a significant effect. Sequencing saturation refers to the sequencing depth of a library. For example, if a library contains a total of 10,000 types of gene sequences, and sequencing is performed on a single lane of the MGI-2000RS sequencer at a sequencing saturation of 17%, it is considered that 17% of the gene types, i.e., 1700 gene types have been detected by the single lane. The remaining sequences that have not been detected can be obtained by additional sequencing, i.e., increasing the number of sequencing lanes. Bin 200 refers to the area defined by 200 DNBs × 200 DNBs on the capture chip. In the present disclosure, probes on the capture chip were generated by DNBs, which, similar to DNBs, were arranged in a dot array with consistent row and column spacing. Bin 200 refers to the square area defined by 200 DNBs horizontally and 200 DNBs vertically. In technical comparisons, a uniform bin 200 is used to compare the effects of different batches of experiments.

Mouse thymus: The test results of the mouse thymus were consistent with those of the mouse brain. That is, when using the method of Comparative Example 1, at a sequencing saturation of 39%, 1900 gene types were detected per bin 200; when using the method of Example 1, at a sequencing saturation of 41%, a total of 6260 gene types were detected per bin 200. The sequencing saturations of 41% and 39% were comparable, indicating basically identical sequencing depths. Under this premise, the gene types captured by the method of Example 1 were 3.3 times those by the method of Comparative Example 1, showing a significant improvement. Additionally, when testing the mouse thymus using the method of Comparative Example 1, diffusion (represented by the gray areas around the tissue in the figure, and darker areas represent less capture and lighter areas represent more capture) was observed, while diffusion was significantly reduced using the method of Example 1, which is an additional finding.

Mouse spleen: The test results of the mouse spleen were consistent with those of the mouse brain and thymus. When using the method of Comparative Example 1, at a sequencing saturation of 81%, 3010 gene types were detected per bin 200. According to estimates, at a sequencing saturation of approximately 40%, approximately 1500 gene types would be detected per bin 200. In contrast, when using the method of Example 1, at a sequencing saturation of 42%, a total of 6340 gene types were detected per bin 200, showing a 4.2-fold improvement in gene capture efficiency with a significant effect.

### Comparative Example 2: T4 ligation followed by RT method

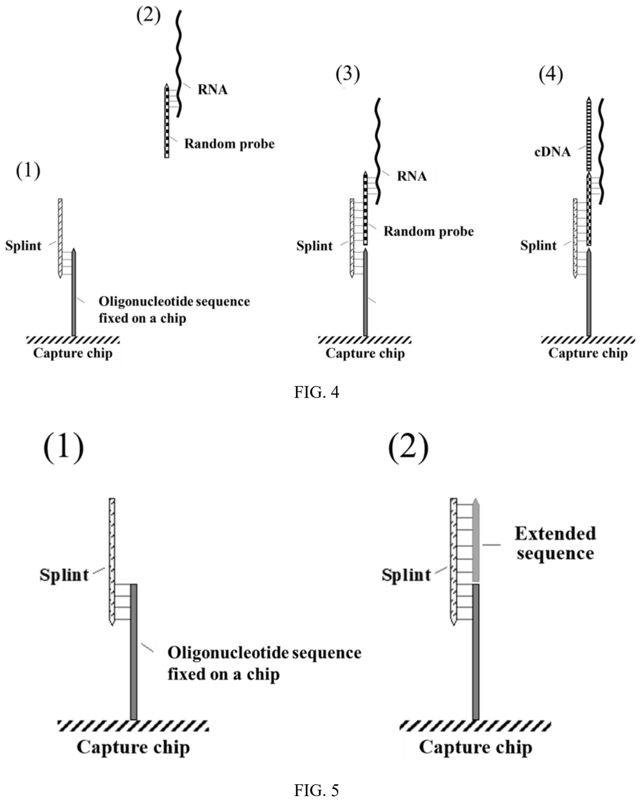

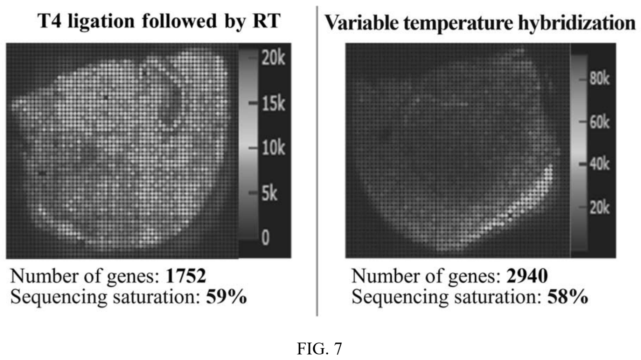

First, the splint was hybridized with the oligonucleotide strand fixed on the chip ((1) in FIG. 4). Then, tissue sections were mounted, fixed, and permeabilized, followed by the addition of random probe 6N to capture RNA ((2) in FIG. 4). Subsequently, T4 ligase was added to ligate the fixed oligonucleotide sequence of the random probe 6N to the oligonucleotide strand on the chip, connecting the random probe 6N that had captured RNA with the oligonucleotide strand on the chip into a single strand ((3) in FIG. 4). Afterward, RT enzyme was added to obtain cDNA by reverse transcription((4) in FIG. 4). Upon testing, it was found that the method resulted in low capture efficiency, as shown in the left panel of FIG. 7. In FIG. 7, mouse brain was used as the experimental material. When using the T4 ligation followed by RT method, the splint was first ligated to the oligonucleotide sequence fixed on the chip, followed by the addition of random probe for RNA capture and reverse transcription, and then T4 ligase was added for ligation. The cDNA libraries obtained in this way were sequenced using the MGI-2000RS sequencer, and at a sequencing saturation of 59%, 1752 gene types were detected per bin 200. In contrast, when using the method of Example 1, at a sequencing saturation of approximately 59%, approximately 4800 gene types were detected per bin 200, showing a 2.7-fold improvement in gene capture efficiency.

When using the variable temperature hybridization method, the splint was first hybridized with the fixed oligonucleotide sequence of the random probe at a high temperature (55°C). Due to the short hybridization length between the splint and the oligonucleotide sequence fixed on the chip, the segment would denature at a high temperature, ensuring that the splint would be fully hybridized with the random probe at a high temperature, followed by returning to a low temperature (around 25°C) to allow the splint to be smoothly hybridized with the oligonucleotide sequence fixed on the chip. The cDNA libraries obtained in this way were sequenced using the MGI-2000RS sequencer, and at a sequencing saturation of 58%, 2940 gene types were detected per bin 200. In contrast, when using the method of Example 1, at a sequencing saturation of approximately 59%, approximately 4800 gene types were detected per bin 200, showing a 1.6-fold improvement in gene capture efficiency.

The specific steps were as follows:
Steps 2.1 to 2.2 of Comparative Example 2 were identical to steps 1.1 to 1.2 of Example 1. Steps 2.3 to 2.6 of Comparative Example 2 were as follows:

### 2.3 Chip processing and tissue mounting

2.3.1 Splint hybridization with capture chip: The capture chip was carefully gripped with forceps and placed into a new 24-well plate. 5 µM splint was dissolved in 5× SSC buffer, mixed well and applied to cover the surface of the capture chip, and hybridized at room temperature for 15 minutes, during which the splint was hybridized with the oligonucleotide sequence fixed on the surface of the capture chip, as shown in (1) in FIG. 4.

2.3.2 Tissue mounting: The chip was washed twice with 400 µL of 0.1× SSC, followed by thorough washing with nuclease-free water (NF-H₂O), and dried at 37°C. A frozen OCT-embedded tissue section of 10 µm thickness was cut and flattened. The capture chip was gripped with forceps and swiftly brought into contact with the frozen section, allowing the tissue section to automatically adhere to the chip. The tissue-mounted chip was then baked at 37°C for 3 to 5 minutes.

### 2.4 Fixation and permeabilization of tissue sections

The baked chip was fixed in a methanol solution pre-chilled to -20°C for 30 minutes. After removal, methanol was air-dried, and permeabilization reagent (prepared by mixing permease with 10 µL of 0.1 N HCl and 90 µL of NF-H₂O) was added. The chip was reacted in an incubator at 37°C for 10 to 13 minutes.

### 2.5 Random probe 6N hybridization and T4 ligation

2.5.1 Random probe 6N hybridization: 5 µM random probe 6N was dissolved in 5× SSC buffer, added with 5% by volume of RI, and mixed well. The random probe solution was applied to the chip and hybridized at room temperature for 15 minutes, during which the random probe 6N was hybridized with RNA in the tissue section for capture ((2) in FIG. 4). The chip was then washed once with 5× SSC and RI.

2.5.2 T4 ligation: T4 ligation reaction buffer (containing 10 mM MgCl₂, 1 mM ATP, 10 mM DTT, pH 7.5 at 25°C) was applied to the washed chip surface, and the mixture was reacted at 16°C overnight, during which, theoretically, the random probe 6N that had captured RNA was ligated to the oligonucleotide sequence on the chip ((3) in FIG. 4). After the reaction was completed, the chip was washed once with 0.1× SSC and RI.

### 2.6 Reverse transcription (RT)

RT reaction buffer (containing RT reagent, 3 mM MgCl₂, 10 mM DTT and 1 mM ATP, pH 8.3 at 25°C; 2.5 µL/ 100 µL of RT enzyme) was applied to the chip, and the mixture was reacted at 42°C for 3 hours. After removal, the chip was washed once with 0.1× SSC and RI.

Steps 2.7 to 2.10 of Comparative Example 2 were identical to steps 1.7 to 1.10 of Example 1.

### Comparative Example 3: Variable temperature hybridization method

After the addition of the random probe 6N to capture RNA and reverse transcribe it into cDNA, a splint was introduced. The splint was first hybridized with the fixed oligonucleotide sequence on the random probe 6N at 55°C, followed by hybridization with the oligonucleotide strand fixed on the chip at room temperature.

During the design of the splint, the hybridization length with the fixed oligonucleotide sequence of the random probe 6N was set to 31 bases (Tm value of 65°C), while the hybridization length with the oligonucleotide strand on the chip was 14 bases (Tm value of 45°C), which enabled a variable temperature hybridization approach, whereby the splint was added and first hybridized with the fixed oligonucleotide sequence on the random probe 6N at a high temperature (55°C). Due to the short hybridization length between the splint and the nucleic acid strand on the chip, the segment would denature at a high temperature of 55°C even if the hybridization occurred, preventing the splint from hybridizing alone with the nucleic acid strand on the chip, thereby avoiding the invalidation of the nucleic acid strand. After the splint was hybridized with the fixed oligonucleotide sequence of the random probe 6N, the chip was washed once with 5× SSC preheated to 55°C to remove excess splint, followed by the addition of 5× SSC, and restored to room temperature, allowing the splint hybridized with random probe 6N to hybridize with the nucleic acid strand on the chip. Subsequently, T4 ligation reaction buffer was added for a ligation reaction. However, upon testing, it was found that the method resulted in unsatisfactory capture efficiency and highly uneven capture distribution, as shown in the right panel of FIG. 7.

Steps 3.1 to 3.4 of Comparative Example 3 were identical to steps 1.1 to 1.4 of Example 1. Steps 3.5 to 3.6 of Comparative Example 3 were as follows:

### 3.5 Random probe 6N hybridization with tissue and reverse transcription (RT)

3.5.1 Random probe 6N hybridization with tissue: 5 µM random probe 6N was dissolved in 5× SSC buffer, added with 5% by volume of RI, and mixed well. The permeabilized tissue section was washed once with 5× SSC and RI, then added with the prepared random probe 6N solution, and hybridized at room temperature for 15 minutes. During the process, RNA in the tissue was captured by a random probe 6N. The chip was then washed once with 5× SSC and RI.

3.5.2 Reverse transcription (RT) reaction: RT reaction buffer (containing RT reagent, 3 mM MgCl₂, 10 mM DTT, 1 mM ATP, pH 8.3 at 25°C; 2.5 µL/100 µL of RT enzyme) was applied to the chip surface. The chip was reacted at 42°C for 3 hours. After removal, the chip was washed once with 0.1× SSC and RI.

### 3.6 Splint hybridization and T4 ligation

3.6.1 Splint hybridization: 5 µM splint was diluted in 5× SSC buffer, added with 5% by volume of RI, and mixed well. The mixture was added to the tissue section washed in the previous step, and hybridized at 55°C for 15 minutes. After removal, the chip was washed once with 5× SSC and RI preheated to 55°C, followed by the addition of 5× SSC buffer, restored to room temperature, and incubated for 15 minutes.

3.6.2 T4 ligation: T4 ligation reaction buffer (containing 10 mM MgCl₂, 1 mM ATP, 10 mM DTT, pH 7.5 at 25°C) was added, and the mixture was reacted at 16°C overnight.

Steps 3.7 to 3.10 of Comparative Example 3 were identical to steps 1.7 to 1.10 of Example 1.

### Comparative Example 4: Method of separate introduction of splint and random probe

In Example 1 of the present disclosure, the steps of RT and T4 ligation were combined, wherein the random probe 6N and splint were first hybridized at a molar ratio of 1:1 to form a mixture ((1) in FIG. 2), followed by RNA capture.

In Comparative Example 4, the splint and random probe 6N were separately introduced in an attempt, where hybridization between the splint and the oligonucleotide strand on the chip could occur((1) in FIG. 5). After adding the reaction system of RT and T4 ligation, a portion of the oligonucleotide strand fixed on the chip would polymerize upward using the splint as a template to form an extended sequence ((2) in FIG. 5). All other steps were the same as in Example 1.

However, the results showed that the method altered the oligonucleotide sequence on the chip. The fixed oligonucleotide sequence of random probes 6N could no longer hybridize with the extended sequence via splint, resulting in the invalidation of the oligonucleotide strand on the chip and the inability to capture RNA from the sample.

Therefore, hybridizing random probe 6N with splint to form a mixture first minimized the probability of hybridization between splint alone and the oligonucleotide strand on the chip, thereby preserving the original hybridization function of the oligonucleotide strand on the chip.

Although the specific embodiments of the present disclosure have been described above, those skilled in the art should understand that these are merely illustrative examples, and various changes or modifications may be made to these embodiments without departing from the principles and essence of the present disclosure. Therefore, the scope of protection of the present disclosure is defined by the appended claims.

## Claims

1. A method for capturing nucleic acid, comprising the following steps:
reverse transcribing an RNA in a sample captured by a random probe into a cDNA, and ligating the cDNA to an oligonucleotide strand by means of splint oligonucleotide hybridization of the random probe and the oligonucleotide strand fixed on a capture chip, wherein the reverse transcription and the ligation are carried out in the same reaction system.

2. The method according to claim 1, wherein the method comprises the following steps:
(1) hybridizing the random probe to the splint sequence; wherein the 5' end of the random probe is partially complementary to the 5' end of the splint sequence;
(2) hybridizing the random probe to the RNA in the sample; wherein the 3' end of the random probe is partially complementary to the 3' end of the RNA;
(3) hybridizing the splint sequence to the fixed oligonucleotide sequence; preferably, the oligonucleotide sequence is fixed on the capture chip;
(4) in the same reaction system, synthesizing cDNA using the RNA in the complex formed by simultaneous hybridization in steps (1), (2), and (3) as a template, and ligating the fixed oligonucleotide sequence to the random probe in the complex, allowing the fixed oligonucleotide sequence, the random probe, and the cDNA to form a long single-stranded nucleotide containing cDNA in the 5' to 3' direction.

3. The method according to claim 2, wherein in step (1), the random probe comprises 6 to 20 N;
in step (3), the capture chip is reacted at 30 to 45°C, such as 37°C, for 3 to 24 hours, such as 3 to 5 hours;
in step (4), a reverse transcriptase and a DNA ligase are present simultaneously in the reaction system;
preferably, the sample comprises a tissue sample.

4. The method according to claim 1, wherein the step further comprises:
(5) aspirating the liquid from the surface of the capture chip, adding a mixture 1 containing a reverse transcriptase and a T4 ligase, and reacting the capture chip in an incubator for 3 to 24 hours.

5. The method according to claim 4, wherein the mixture 1 contains a reverse transcription reagent, 3 to 25 mM of MgCl₂, 1 to 50 mM of DTT, and 1 mM or more of ATP;
the volume ratio of the reverse transcriptase to the T4 ligase is 2:1 or 1:1; and/or,
the incubator has a temperature of 35 to 42°C.

6. The method according to claim 4, wherein the mixture has a pH of 8.3.

7. The method according to claim 4, wherein before step (5), the method further comprises:
(i) while fixing a tissue section to the capture chip, preparing a probe hybridization solution and mixing with 5× SSC, performing a hybridization in an incubator at 50 to 60°C, such as 55°C, for 5 to 30 minutes, such as 10 minutes, to form a primer hybridization mixture, removing and cooling the primer hybridization mixture, and adding 2 to 15% by volume, such as 5% by volume of RNase inhibitor;
(ii) removing the capture chip and aspirating the liquid from the chip surface, washing the capture chip with a mixture 2, adding the primer hybridization mixture to the chip surface, performing a hybridization at room temperature for 15 to 60 minutes, and washing the capture chip again with a mixture 2;
the mixture 2 contains 5× SSC and RNase inhibitor;
the probe hybridization solution contains the random probe and splint.

8. The method according to claim 5, wherein the tissue section is a frozen OCT tissue section;
the probe hybridization solution contains the random probe and splint with a molar ratio of 1:1;
the cooling comprises placing the mixture on ice and/or placing the mixture at room temperature until the mixture cools to room temperature, and/or
the volume ratio of 5× SSC to RNase inhibitor in the mixture 2 is (5 to 30):1, such as 19:1.

9. The method according to claim 7 or 8, wherein the sequence of the random probe is as shown in SEQ ID NO: 2; the sequence of the splint is as shown in SEQ ID NO: 3.

10. The method according to claim 1, wherein the method further comprises the following steps:
(a) after completing step (ii), removing the tissue sample from the capture chip;
(b) recovering cDNA from the capture chip; optionally further comprising the steps of cDNA amplification and purification.

11. The method according to any one of claims 1 to 10, wherein the capture chip comprises a capture probe, wherein the capture probe comprises spatial barcode location information and a fixed oligonucleotide sequence; the fixed oligonucleotide sequence is hybridized to the 3' end of the splint.

12. The method according to claim 11, wherein the capture probe is 5'-Nm-SEQ ID NO: 1-3', wherein m is 8 to 30.

13. The method according to claim 12, wherein the m is 25 or more.

14. A high-throughput sequencing method, comprising the following steps:
(I) obtaining a long single-stranded nucleotide containing cDNA to be sequenced according to the method according to any one of claims 1 to 13;
(II) constructing a cDNA library;
(III) sequencing the library and analyzing the obtained data.

15. The method according to claim 14, wherein step (I) is followed by the steps of cDNA fragmentation, amplification, and purification; and/or, in step (II), the cDNA library is circularized to obtain a circularized library of DNB.
